# EUROPEAN PATENT APPLICATION

(11) **EP 2 719 717 A1**
(43) Date of publication of application: **16.04.2014**
(21) Application number: 13188248.2
(22) Date of filing: 11.10.2013
(51) Int. Cl.: C08J 5/18, A61L 31/14, A61K 9/70, C08L 101/16

(54) **Compositions and methods of forming films for improved drug delivery**

(30) Priority: 12.10.2012 US 201261712839 P; 17.09.2013 US 201314028945
(71) Applicant: Covidien LP, Mansfield, MA 02048 (US)
(72) Inventor: Witherell, Ryan, Glastonbury, CT Connecticut 06033 (US); Elachchabi, Amin, Hamden, CT Connecticut 06518 (US); Stopek, Joshua, Minneapolis, MN Minnesota 55419-5240 (US); Ebersole, Garrett, New Haven, CT Connecticut 06511 (US)
(74) Representative: Maschio, Antonio

(57) **Abstract**

The present disclosure relates to compositions and methods for fabricating films for the delivery of a therapeutic agent containing at least one polymer and at least one therapeutic agent in a composition including a decreased alcohol fraction for providing smoother films and desired release characteristics. Drug-eluting films are also disclosed.

## Description

### CROSS-REFERENCE TO RELATED APPLICATIONS

This application claims the benefit of and priority to U.S. Provisional Patent Application No. 61/712,839, filed October 12, 2012, the entire disclosure of which is incorporated by reference herein.

### BACKGROUND

### Technical Field

The present disclosure describes compositions and methods of forming films for delivery of a therapeutic agent, and in embodiments, compositions and methods of forming smooth films which yield uniform, linear release profiles of the therapeutic agent.

### Background of Related Art

Biodegradable controlled release systems for therapeutic agents are useful because they obviate the need to remove the drug-depleted device. A well-designed controlled release formulation should be capable of presenting a linear release profile of a therapeutically effective amount of a drug in vivo.

Typically, a controlled release system includes a therapeutic agent dispersed in a polymer matrix. Therapeutic agents having poor solubility in an aqueous solution, such as hydrophobic therapeutic agents, are frequently employed as their hydrochloride salts to allow for water or alcohol solubility. Water-soluble therapeutic agents, on the other hand, offer limited solubility in the organic systems particularly useful with hydrophobic or water-insoluble carriers, e.g., hydrophobic polymers. Limited solubility of the highly water-soluble therapeutic agents may lead to limited therapeutic payload in the implantable device.

Various approaches have been used to provide a desired release profile including: a mix of two different polymers; a polymer that swells to form a hydrogel; or a polymer with an increased number of endgroups with acidic function or other hydrophilic end groups. To form a controlled release film, the foregoing polymer systems typically involve solvent blending using an organic solvent such as ethanol. However, solvent residues in the films may create the potential for drug or polymer degradation, and may affect film surface smoothness thereby adversely impacting the characteristics of the film. Thus, controlled release films having smooth surfaces with linear release profiles would be desirable.

### SUMMARY

The present disclosure relates to compositions and methods utilized in the fabrication of controlled release films. Specifically, the present disclosure provides compositions and methods of forming films for the delivery of one or more therapeutic agents, the films including at least one polymer, at least one therapeutic agent, an alcohol, and at least one chlorinated hydrocarbon. The films having smooth surfaces and linear release profiles.

Methods of forming films may include providing a first therapeutic solution containing at least one alcohol, at least one chlorinated hydrocarbon and at least one therapeutic agent, wherein the weight ratio of the alcohol to the chlorinated hydrocarbon ranges from about 1:1 to about 1:20; providing a second polymer solution containing at least one polymer in a solvent; combining the first and second solution to form a film-forming composition; and forming at least one layer of a film with the film-forming composition. In embodiments, the therapeutic solution may further include a polymer.

Methods of forming a film may also include providing a composition containing at least one alcohol, at least one chlorinated solvent, at least one therapeutic agent, and at least one polymer, wherein the weight ratio of the alcohol to the chlorinated solvent ranges from about 1:1 to about 1:20; and forming at least one layer of the film with the composition.

Controlled release films formed by the methods of the present disclosure are provided.

Compositions for forming a controlled release film are also provided, including at least one therapeutic agent; at least one polymer; at least one alcohol; and at least one chlorinated hydrocarbon; wherein the weight ratio of the alcohol to the chlorinated hydrocarbon may range from about 1:1 to about 1:20, in some embodiments from about 1:3 to about 1:15, and in some embodiments from about 1:5 to about 1:10.

In embodiments, the alcohol may be methanol. In embodiments, the at least one chlorinated hydrocarbon may be dichloromethane. In embodiments, the therapeutic agent may be an anesthetic in its salt form such as bupivacaine HCL.

In some embodiments, the films may be multilayered and may include at least a first layer containing at least one polymer solution and at least a second layer containing at least one therapeutic agent solution. In some embodiments, the films may be multilayered and may include a core or inner layer formed from a composition containing at least one copolymer with at least one therapeutic agent. Barrier layers may also be positioned on any side of the core or inner layer, such as on top, bottom or the side of the inner layer. In alternative embodiments, the films may be a single layer and may include at least one polymer and at least one therapeutic agent.

### BRIEF DESCRIPTION OF THE DRAWINGS

FIG. 1 is a scanning electron microscope (SEM) image (Mag.=150X) of a cross-sectional view of a multi-laminar film fabricated from a composition containing ethanol (EtOH) at a 1:1 ratio with dichloromethane (MeCl₂) as described in a comparative example of the present disclosure;

FIG. 2A is a SEM image (Mag.=100X) of a cross-sectional view of a multi-laminar film fabricated from a composition with a 1:1 ratio of methanol (MeOH) to MeCl₂ in accordance with at least one embodiment described herein;

FIG. 2B is a SEM image (Mag.=300X) of the cross-sectional view of the multi-laminar film illustrated in FIG. 2A;

FIG. 2C is a SEM image (Mag.=100X) of a cross-sectional view of a multi-laminar film fabricated from a composition with a 1:4 ratio of MeOH to MeCl₂ in accordance with at least one embodiment described herein;

FIGS. 2D and 2E are SEM images (Mag=600X) of the cross-sectional views of the multi-laminar film illustrated in FIG. 2C;

FIG. 3A is a SEM image (Mag=100X) of a cross-sectional view of a multi-laminar film fabricated from a composition with a 1:9 ratio of MeOH to MeCl₂ in accordance with at least one embodiment described herein;

FIG. 3B is a closer SEM image (Mag=600X) of the cross-sectional view of the multi-laminar film illustrated in FIG. 3A;

FIG. 4 is a graph illustrating release profiles of multi-laminar films prepared with a 1:1 MeOH : MeCl₂ solution, a 1:4 MeOH : MeCl₂ and a 1:9 MeOH : MeCl₂ in accordance with at least one embodiment described herein;

FIG. 5 is a diagrammatic illustration of a barrier layer realized as a multi-laminar film, according to at least one embodiment described herein; and

FIGS. 6A, 6B, and 6C are cross-sectional views of multi-laminar films in accordance with at least one embodiment described herein.

### DETAILED DESCRIPTION

The present disclosure provides compositions for forming implantable films for the delivery a therapeutic agent and methods of forming such films. The methods described herein provide implantable films which display a generally smooth outer surface and a linear release profile of the therapeutic agent.

The term "linear release profile" refers to the delivery of a fixed amount of the therapeutic agent or drug per unit time over an administration period. In embodiments, the films described herein release the therapeutic agent at a uniform rate during the administration period, independent of the concentration rate of the therapeutic agent in the film. In embodiments, the release profile may be defined by a plot of the cumulative drug released versus the time during which the release takes place in which the linear least squares fit of such a release profile plot has a correlation coefficient, r² (the square of the correlation coefficient of the least squares regression line), of greater than 0.92 for data time points after the first day of delivery.

A linear release profile maybe clinically significant in that it may allow for release of a prescribed dosage of the therapeutic agent at a uniform rate over an extended length of time as compared to agents which are typically delivered orally and/or intravenously. This controlled release may be essential to maximizing the effectiveness of a therapeutic agent while minimizing potential side effects. In addition, it can reduce the dosing frequency from once every 2-8 hours to simply once upon implantation. In embodiments, the delivery of the therapeutic agent may range from hours to days to weeks, and more particularly, from 24 hours to 1 week, and more particularly from 24-72 hours.

The terms "generally smooth outer surface" and/or "surface smoothness" refer to an outer surface of the films described herein that is free of microscopic pits greater than about 10 micrometers in depth. In embodiments, the surface roughness of the films ranges from about 0.001 to about 10 micrometers. In embodiments, the surface roughness of the films ranges from about 0.1-3 micrometers, and more particularly, less than about 2 micrometers, e.g., about 1 micrometer, about 0.5 micrometers, and about 0.25 micrometers.

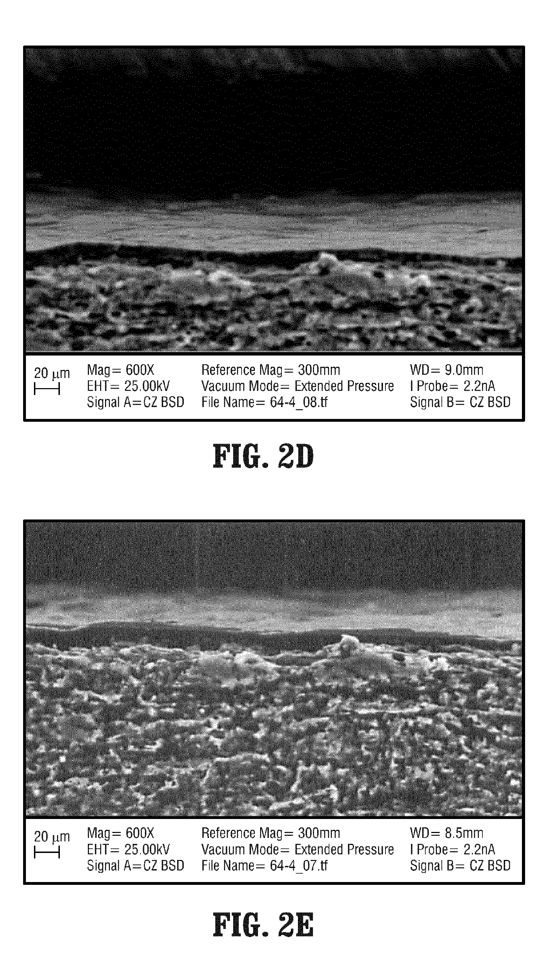

As illustrated in Fig. 2E, in embodiments, the surface smoothness may be measured by extending an imaginary line at the highest peak of the outer surface which extends generally parallel following the contour of the outer surface of the film and measuring the area (Rₐ) between the extended parallel line and the outer surface of the film. In embodiments, at a magnification of 600x, the average Rₐ value is less than 4,000 µm², and in particular embodiments less than 2,500 µm².

In embodiments, the implantable films may be made from compositions which include at least one polymer and at least one therapeutic agent combined with a chlorinated hydrocarbon and an alcohol. Additional biocompatible materials and/or optional ingredients may also be included.

In embodiments, methods of the present disclosure may also include providing a composition containing an alcohol, at least one chlorinated hydrocarbon, at least one therapeutic agent, and at least one polymer, wherein the ratio of the alcohol to the at least one chlorinated hydrocarbon is from about 1:1 to about 1:20; and forming at least one layer of the film with the composition.

In embodiments, the present disclosure describes methods for forming the implantable films which include: providing a first solution containing an alcohol, at least one chlorinated hydrocarbon or solvent and at least one therapeutic agent, wherein the ratio of the alcohol to the at least one chlorinated hydrocarbon is from about 1:1 to about 1:20; providing a second solution containing at least one polymer; combining the first and second solution to form a film-forming composition; and forming at least one layer of the film with the film-forming composition. Polymers

The term "polymer" as used herein, refers to a series of repeating monomeric units that have been cross-linked or polymerized. Any suitable polymer can be used to for compositions and films described herein. It is possible that the polymers may comprise two, three, four or more different polymers. In some embodiments only one polymer is used. In certain embodiments a combination of two or more polymers is used. Combinations of polymers can be in varying ratios, to provide compositions with differing properties.

Polymers useful in the compositions, films and methods of the present disclosure include, for example, bioabsorbable and non-bioabsorbable polymers. The terms "bioabsorbable," "biodegradable," "bioerodible," "bioresorbable," and "resorbable" are art-recognized synonyms. The terms "non-bioabsorbable," "non-biodegradable," "non-bioerodible," "non-bioresorbable," and "non-resorbable" are also art-recognized synonyms. These terms may be used herein interchangeably. Bioabsorbable polymers typically differ from non-bioabsorbable polymers in that the former may be absorbed (e.g., degraded) during use. Those of skill in the art of polymer chemistry will be familiar with the different properties of polymeric compounds.

In various embodiments, the compositions and/or films include a bioabsorbable polymer that is capable of resorbtion in at least one of: about 1 day, about 3 days, about 5 days, about 7 days, about 14 days, about 3 weeks, about 4 weeks, about 45 days, about 60 days, about 90 days, about 180 days, about 6 months, about 9 months, about 1 year, about 1 to about 2 days, about 1 to about 5 days, about 1 to about 2 weeks, about 2 to about 4 weeks, about 45 to about 60 days, about 45 to about 90 days, about 30 to about 90 days, about 60 to about 90 days, about 90 to about 180 days, about 60 to about 180 days, about 180 to about 365 days, about 6 months to about 9 months, about 9 months to about 12 months, about 9 months to about 15 months, and about 1 year to about 2 years.

Examples of suitable polymers that may be used in the present disclosure include, but are not limited to polycarboxylic acids, cellulosic polymers, proteins, polypeptides, polyvinylpyrrolidone, maleic anhydride polymers, polyamides, polyvinyl alcohols, polyethylene oxides, glycosaminoglycans, polysaccharides, polyesters, aliphatic polyesters, polyurethanes, polystyrenes, copolymers, silicones, silicone containing polymers, polyalkyl siloxanes, polyorthoesters, polyanhydrides, copolymers of vinyl monomers, polycarbonates, polyethylenes, polypropylenes, polylactic acids, polylactides, polyglycolic acids, polyglycolides, polylactide-co-glycolides, polycaprolactones, poly(e-caprolactone)s, polyglycolides-co-caprolactones, polyhydroxybutyrate valerates, polyacrylamides, polyethers, polyurethane dispersions, polyacrylates, acrylic latex dispersions, polyacrylic acid, polyalkyl methacrylates, polyalkylene-co-vinyl acetates, polyalkylenes, aliphatic polycarbonates polyhydroxyalkanoates, polytetrahalooalkylenes, poly(phosphasones), polytetrahalooalkylenes, poly(phosphasones), and mixtures, combinations, and copolymers thereof.

The polymers may be natural or synthetic in origin, including gelatin, chitosan, dextrin, cyclodextrin, poly(urethanes), poly(siloxanes) or silicones, poly(acrylates) such as poly(butyl methacrylate), and poly(2-hydroxy ethyl methacrylate), poly(vinyl alcohol) poly(olefins) such as poly(ethylene), poly(isoprene), halogenated polymers such as poly(tetrafluoroethylene)--and derivatives and copolymers such as those commonly sold as Teflon® products, poly(vinylidine fluoride), poly(vinyl acetate), poly(vinyl pyrrolidone), poly(acrylic acid), polyacrylamide, poly(ethylene-co-vinyl acetate), poly(ethylene glycol), poly(propylene glycol), poly(methacrylic acid); etc.

In particular embodiments, the polymers used to form the compositions and/or films described herein include the following, combinations, polymers, copolymers and derivatives of the following: polylactides (PLA), polyglycolides (PGA), polylactide-co-glycolides (PLGA), polyanhydrides, polyorthoesters, poly(dl-lactide), poly(l-lactide), poly(dioxanone), poly(glycolide-co-trimethylene carbonate), poly(l-lactide-co-glycolide), poly(dl-lactide-co-glycolide), poly(l-lactide-co-dl-lactide), poly(glycolide-co-trimethylene carbonate-co-dioxanone), poly(glycolide-co-caprolactone), poly(glycolide-co-έ-caprolactone), and combinations thereof.

In some embodiments, the polymer may include a copolymer glycolide and/or polyglycolide and έ-caprolactone or poly(έ-caprolactone). The glycolide and/or polyglycolide portion of the copolymer may represent from about 5% to about 95% of the copolymer. In embodiments, the glycolide and/or polyglycolide portion of the copolymer may represent from about 10% to about 75% of the copolymer, and more particularly from about 5% to about 25% of the copolymer. The έ-caprolactone or poly(έ-caprolactone) portion of the copolymer may represent from about 5% to about 95% of the copolymer and in some embodiments from about 75% to about 95% of the copolymer.

The term "copolymer" as used herein refers to a polymer being composed of two or more different monomers. A copolymer may also and/or alternatively refer to random, block, graft, branched, copolymers known to those of skill in the art.

### Therapeutic Agent(s)

In embodiments, the methods include providing a composition or solution containing at least one therapeutic agent. The term "therapeutic agent", as used herein, is used in its broadest sense and includes any substance or mixture of substances that provides a beneficial, therapeutic, pharmacological, and/or prophylactic effect. The agent may be a drug which provides a pharmacological effect.

The term "drug" is meant to include any agent capable of rendering a therapeutic affect, such as, anti-adhesives, antimicrobials, analgesics, antipyretics, anesthetics (e.g. local and systemic), antiepileptics, antihistamines, anti-inflammatories, cardiovascular drugs, diagnostic agents, sympathomimetics, cholinomimetics, antimuscarinics, antispasmodics, hormones, growth factors, muscle relaxants, adrenergic neuron blockers, antineoplastics, immunogenic agents, immunosuppressants, gastrointestinal drugs, diuretics, steroids, lipids, lipopolysaccharides, polysaccharides, platelet activating drugs, clotting factors, and enzymes. It is also intended that combinations of agents may be used.

Other therapeutic agents, which may be included as a drug include: anti-fertility agents; parasympathomimetic agents; psychotherapeutic agents; tranquilizers; decongestants; sedative hypnotics; sulfonamides; sympathomimetic agents; vaccines; vitamins; antimalarials; antimigraine agents; anti-parkinson agents such as L-dopa; anti-spasmodics; anticholinergic agents (e.g., oxybutynin); antitussives; bronchodilators; cardiovascular agents, such as coronary vasodilators and nitroglycerin; alkaloids; analgesics; narcotics such as codeine, dihydrocodeinone, meperidine, morphine and the like; non-narcotics, such as salicylates, aspirin, acetaminophen, d-propoxyphene and the like; opioid receptor antagonists, such as naltrexone and naloxone; anti-cancer agents; anti-convulsants; anti-emetics; antihistamines; anti-inflammatory agents, such as hormonal agents, hydrocortisone, prednisolone, prednisone, non-hormonal agents, allopurinol, indomethacin, phenylbutazone and the like; prostaglandins and cytotoxic drugs; chemotherapeutics; estrogens; antibacterials; antibiotics; anti-fungals; anti-virals; anticoagulants; anticonvulsants; antidepressants; and immunological agents.

Other examples of suitable agents, which may be included in the films described herein include, for example, viruses and cells; peptides, polypeptides and proteins, as well as analogs, muteins, and active fragments thereof; immunoglobulins; antibodies; cytokines (e.g., lymphokines, monokines, chemokines); blood clotting factors; hemopoietic factors; interleukins (e.g., IL-2, IL-3, IL-4, IL-6); interferons (e.g., β-IFN, α-IFN and γ-IFN); erythropoietin; nucleases; tumor necrosis factor; colony stimulating factors (e.g., GCSF, GM-CSF, MCSF); insulin; anti-tumor agents and tumor suppressors; blood proteins such as fibrin, thrombin, fibrinogen, synthetic thrombin, synthetic fibrin, synthetic fibrinogen; gonadotropins (e.g., FSH, LH, CG, etc.); hormones and hormone analogs (e.g., growth hormone); vaccines (e.g., tumoral, bacterial and viral antigens); somatostatin; antigens; blood coagulation factors; growth factors (e.g., nerve growth factor, insulin-like growth factor); bone morphogenic proteins; TGF-B; protein inhibitors; protein antagonists; protein agonists; nucleic acids such as antisense molecules, DNA, RNA, and RNAi; oligonucleotides; polynucleotides; and ribozymes.

Some specific non-limiting examples of water-soluble drugs that may be used in the present films include, bupivacaine, lidocaine, tetracaine, procaine, dibucaine, sirolimus, taxol, chlorhexidine, polyhexamethylene, thiamylal sodium, thiopental sodium, ketamine, flurazepam, amobarbital sodium, phenobarbital, bromovalerylurea, chloral hydrate, phenytoin, ethotoin, trimethadione, primidone, ethosuximide, carbamazepine, valproate, acetaminophen, phenacetin, aspirin, sodium salicylate, aminopyrine, antipyrine, sulpyrine, mepirizole, tiaramide, perixazole, diclofenac, anfenac, buprenorphine, butorphanol, eptazocine, dimenhydrinate, difenidol, dl-isoprenaline, chlorpromazine, levomepromazine, thioridazine, fluphenazine, thiothixene, flupenthixol, floropipamide, moperone, carpipramine, clocapramine, imipramine, desipramine, maprotiline, chlordiazepoxide, clorazepate, meprobamate, hydroxyzine, saflazine, ethyl aminobenzoate, chlorphenesin carbamate, methocarbamol, acetylcholine, neostigmine, atropine, scopolamine, papaverine, biperiden, trihexyphenidyl, amantadine, piroheptine, profenamine, levodopa, mazaticol, diphenhydramine, carbinoxamine, chlorpheniramine, clemastine, aminophylline, choline, theophylline, caffeine, sodium benzoate, isoproterenol, dopamine, dobutamine, propranolol, alprenolol, bupranolol, timolol, metoprolol, procainamide, quinidine, ajmaline, verapamil, aprindine, hydrochlorothiazide, acetazolamide, isosorbide, ethacrynic acid, captopril, enalapril, delapril, alacepril, hydralazine, hexamethonium, clonidine, bunitrolol, guanethidine, bethanidine, phenylephrine, methoxamine, diltiazem, nicorandil, nicametate, nicotinic-alcohol tartrate, tolazoline, nicardipine, ifenprodil, piperidinocarbamate, cinepazide, thiapride, dimorpholamine, levallorphan, naloxone, hydrocortisone, dexamethasone, prednisolone, norethisterone, clomiphene, tetracycline, methyl salicylate, isothipendyl, crotamiton, salicylic acid, nystatin, econazole, cloconazole, vitamin B₁ , cycothiamine, vitamin B_{2,} vitamin B_{3,} vitamin B_{5,} vitamin B₆, vitamin B_{7,} vitamin B_{9,} vitamin B_{12,} vitamin C_{,} nicotinic acid, folic acid, nicotinamide, calcium pantothenate, pantothenol, panthetin, biotin, ascorbic acid, tranexamic acid, ethamsylate, protamine, colchicine, allopurinol, tolazamide, glymidine, glybuzole, metoformin, buformin, orotic acid, azathioprine, lactulose, nitrogen mustard, cyclophophamide, thio-TEPA, nimustine, thioinosine, fluorouracil, tegafur, vinblastine, vincristine, vindesine, mitomycin C, daunorubicin, aclarubicin, procarbazine, cisplatin, methotrexate, benzylpenicillin, amoxicillin, penicillin, oxycillin, methicillin, carbenicillin, ampicillin, cefalexin, cefazolin, erythromycin, kitasamycin, chloramphenicol, thiamphenicol, minocycline, lincomycin, clindamycin, streptomycin, kanamycin, fradiomycin, gentamycin, spectinomycin, neomycin, vanomycin, tetracycline, ciprofloxacin, sulfanilic acid, cycloserine, sulfisomidine, isoniazid, ethambutol, acyclovir, gancyclovir, vidabarine, azidothymidine, dideoxyinosine, dideoxycytosine, morphine, codeine, oxycodone, hydrocodone, cocaine, pethidine, fentanyl, polymeric forms of any of the above drugs and any combinations thereof.

In embodiments, the water or alcohol soluble drug may be utilized in its salt form, i.e., bupivacaine hydrochloride. In embodiments, the therapeutic agent may include an anesthetic, i.e., bupivacaine, lidocaine, benzocaine, and the like.

### Chlorinated Hydrocarbons and Alcohols

In embodiments, methods of the present disclosure may include providing a composition or solution containing at least one chlorinated hydrocarbon and at least one alcohol. In embodiments, the chlorinated hydrocarbon may have a lower boiling point than the alcohol. Some non-limiting examples of suitable chlorinated hydrocarbons include solvents such as methyl chloride, methylene chloride, dichloromethane, chloroform, carbon tetrachloride, and combinations thereof. In embodiments, the chlorinated hydrocarbon may be dichloromethane.

The compositions or solutions described herein further include an alcohol in combination with the chlorinated hydrocarbon and the therapeutic agents. In embodiments, the alcohol may include methanol. In embodiments, the alcohol may be any alcohol suitable for forming smooth films when combined with the chlorinated hydrocarbon and which display a linear release profile of the therapeutic agent.

In embodiments, processes of the present disclosure for fabricating a film capable of eluting the therapeutic agent or drug may include a ratio of alcohol to the chlorinated hydrocarbon or solvent of from about 1:1 to about 1:20, in embodiments from about 1:3 to about 1:10, in still other embodiments from about 1:4 to about 1:9, by weight. In certain embodiments, the ratio of alcohol to the chlorinated hydrocarbon or solvent is about 1:4. In certain embodiments, the ratio of alcohol to the chlorinated hydrocarbon or solvent is about 1:9.

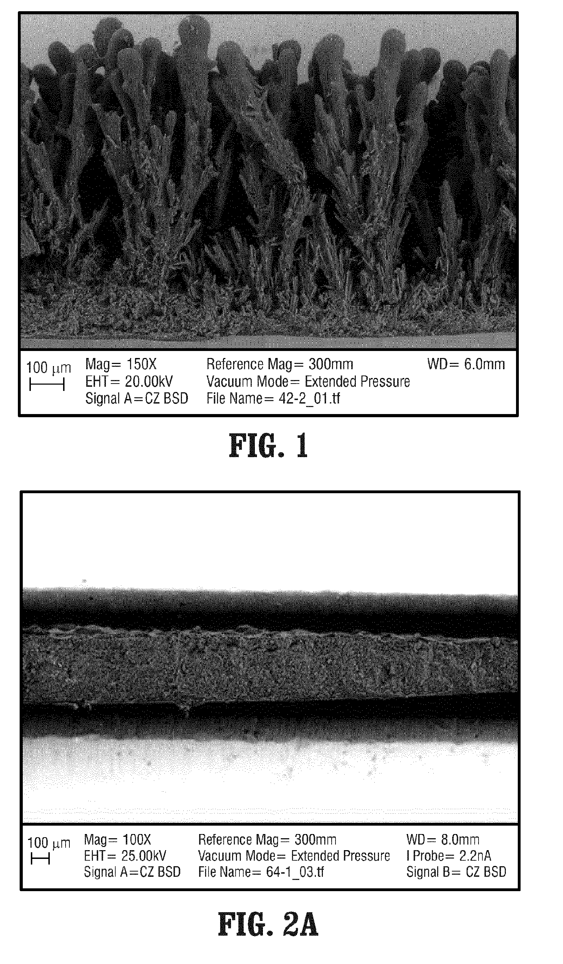

In embodiments, the processes of the present disclosure yield smoother films and films which have linear release profiles. However, as illustrated in Fig. 1, utilization of ethanol to dissolve the therapeutic agents in a chlorinated hydrocarbon based composition at a 1:1 ratio, may yield uneven, rough films with non-linear release characteristics. Accordingly, the type and amount of alcohol combined at a certain ratio with a chlorinated hydrocarbon affects a film's surface smoothness and ability to provide a linear release profile.

In addition, the type and amount of alcohol used to form the compositions, solutions and/or films described herein may affect the amount of alcohol remaining in the film following formation which at certain levels may be toxic. Thus, by combining the alcohol with the chlorinated hydrocarbon or solvent, the amount of alcohol is reduced thereby lowering the potential for toxic levels of alcohol remaining in the films following formation. Still further, the films of the present disclosure may be more stable when exposed to elevated temperatures such as those in an ethylene oxide sterilization cycle.

In embodiments, the evaporation rate of the alcohol/chlorinated hydrocarbon containing solution may be faster than the evaporation rate of a solution containing only one of the chlorinated hydrocarbon and/or alcohol. It is envisioned that a solution containing the therapeutic agent which evaporates quickly and/or at a faster rate, is likely to prevent the therapeutic agent from crystallizing during the formation of the film and ultimately may produce a smooth outer surface.

### Methods of Forming the Films

In embodiments, methods of the present disclosure may include providing a composition containing an alcohol, at least one chlorinated hydrocarbon, at least one therapeutic agent, and at least one polymer, wherein the ratio of the alcohol to the at least one chlorinated hydrocarbon is from about 1:1 to about 1:20; and forming at least one layer of the film with the composition.

In embodiments, the present disclosure describes methods for forming the implantable films which include: providing a first therapeutic solution containing an alcohol, at least one chlorinated hydrocarbon or solvent and at least one therapeutic agent, wherein the ratio of the alcohol to the at least one chlorinated hydrocarbon is from about 1:1 to about 1:20; providing a second polymer solution containing at least one polymer; combining the first and second solution to form a film-forming composition; and forming at least one layer of the film with the film-forming composition. In embodiments, the therapeutic solution may also include at least one polymer.

The polymer solution, including solutions, suspensions, emulsions, dispersions and the like, includes at least one polymer and a suitable solvent. Some non-limiting examples of solvents suitable for forming the polymer solutions may include methylene chloride, chloroform, N-methylpyrrolidone, tetrahydrofuran, dimethylformamide, methanol, hexanes, acetone and combinations thereof. The polymer may represent from about 1.0% to about 25% (w/w) in the solution.

In embodiments, the solvent used for the polymer solution may be the same solvent used to form the therapeutic solution, e.g., a combination of a chlorinated hydrocarbon and alcohol at a ratio ranging from about 1:1 to about 1:20. In embodiments, the solvent used for the polymer solution may not be the same solvent used to form the therapeutic agent solution. For example, methanol may be combined with dichloromethane and bupivacaine HCL to form the therapeutic solution, and the polymer may be dissolved in dichloromethane to form the polymer solution. In addition, the polymeric solutions and/or the therapeutic solutions may include at least one optional ingredient such as surfactants, emulsifiers, viscosity enhancers, dyes, pigments, fragrances, pH modifiers, wetting agents, plasticizers, antioxidants, foaming agents, amphiphilic compounds, and the like. For example, the solutions may include a foaming agent to induce porosity of the film. In another example, the solutions may include amphiphilic compounds to increase water diffusion of the film. The optional ingredients may represent up to about 10% (w/w) of the polymer solution.

The therapeutic agent may form a therapeutic solution at a concentration ranging from about 1 microgram/mL to about 1gram/mL. In certain embodiments, the concentration of the therapeutic solution may range from about 1 milligram/mL to about 500 milligrams/mL. In still other embodiments, the concentration of the therapeutic solution may range from about 10 mg/mL to about 300 mg/mL. By solution, the therapeutic preparation is intended to include suspensions, emulsions, dispersions, and the like.

In embodiments, the processes of the present disclosure may further include forming at least one layer of the film with the film-forming composition. In some embodiments, the processes may further include forming inner, core layers including at least one layer of the first therapeutic solution and at least one layer of the second polymer solution followed by the step of forming at least one top barrier layer with at least one of the first and second solution or the film forming composition. In alternative embodiments, the inner, core layers may include the film-forming composition containing the at least one polymer, at least one therapeutic agent, alcohol and chlorinated solvent.

In embodiments, at least one layer of a film may be formed by spray coating. In embodiments, the spray coating may be ultrasonic spray coating. The composition, therapeutic solution and/or the polymer solution may be passed either in combination or separately through an ultrasonic spray nozzle to form layers of the films described herein. Ultrasonic sprayers include ultrasonic spray nozzles which may be used to generate vibrations leading to atomization of the solutions. The sprayer body consists of three principal sections: front horn, the atomizing section; rear horn, the rear section; and, a section consisting of a pair of disc-shaped piezoelectric transducers. Working in unison, these three elements provide means for creating the vibration required to atomize the solutions delivered to the nozzle surface. The compositions and/or solutions enter through a fitting on the rear, passes through the tube and then the central axis of the front horn. Finally, the solution reaches the atomizing surface of the nozzle where atomization takes place. Piezoelectric transducers convert electrical energy provided by an external power source into high-frequency mechanical motion or vibration. The solution absorbs the underlying vibration energy and generates capillary waves. When the amplitude of the capillary waves exceeds a critical value, the waves collapse ejecting small droplets of the solutions.

The ultrasonic sprayer nozzle may include a variety of controls which may be adjusted to alter the characteristics of the films described herein. Some non-limiting examples include: vibration frequency, operational power; solution flow rates, nozzle speed, and length of movement of the nozzle. In forming the films described herein, the sprayer nozzle may vibrate at a frequency ranging from about 20kHz to about 180 kHz and may operate at a power ranging from about .5 to about 15 watts. In some embodiments, the sprayer nozzle may vibrate at a frequency of about 48kHz and operate at a power of about 2 watts.

In certain embodiments, the ultrasonic spray nozzles may be movable. The nozzle may move at a speed ranging from about 10 mm/sec to about 200 mm/sec. In other embodiments, the nozzle speed may range from about 50 mm/sec to about 150 mm/sec. In addition, the height of the movable nozzles may range from about 30 mm to about 60 mm from the inert substrate.

Also, the flow rate of the solutions passed through the sprayer nozzle may vary within the range of about 0.1 mL/min to about 5 mL/min. In embodiments, the flow rate of the solutions may be within the range of about 0.5 mL/min and 2.0 mL/min. The flow rate may be different for each of the polymer solution and the therapeutic solutions. It is envisioned that each of the sprayer controls may be individually adjusted for each of the different compositions and/or solutions being passed therethrough.

Once sprayed, the films may be dried at ambient (25°C) or elevated temperatures and humidity. Depending on film thickness/number of layers, the films may dry in from about 1 minute to about 24 hours.

As mentioned above, the process of the present disclosure may include sterilizing the film formed by any means known in the art. For example, the film may be sterilized with ethylene oxide.

In embodiments, drug-eluting films may be formed from the processes of the present disclosure.

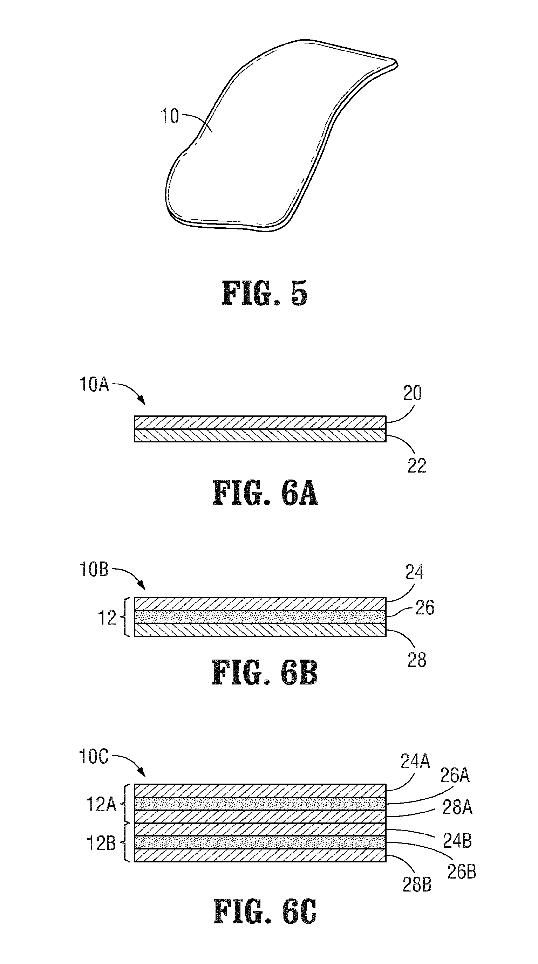

In embodiments, the films described herein may comprise several layers, creating a multi-laminate film. Films of the present disclosure may comprise continuous or discontinuous films. For example, a continuous film as shown in FIGS. 6A-6C described in more detail below may include a single, uninterrupted layer. In another embodiment, films or layers of films may be discontinuous (not shown). In embodiments, individual layers of the multilayer films may be continuous or discontinuous.

Multi-laminate films may comprise similar or different materials. The multilayered film may be created by stacking or combining several layers of films containing the polymer and therapeutic agent. In other embodiments, the multilayered films include a first layer containing a degradable polymer and a second layer containing a therapeutic agent. It may be useful to have an exposed layer of a therapeutic agent for providing unidirectional drug delivery.

FIGS. 6A, 6B, and 6C illustrate cross sectional views of multilayer films. For example, in FIG. 6A, film 10A is shown including first layer 20 and second layer 22. First layer 20 includes at least one polymer and second layer 22 includes at least one therapeutic agent. In some embodiments, the therapeutic agent and/or the polymer may be combined in either first layer 20 or second layer 22.

The multi-laminate films may also comprise different polymer chain orientations, i.e., they may have anisotropic properties, which when combined (optionally at various orientations relative to one another) create a stronger implant and desired drug release profiles.

In other embodiments, the films may include a tri-layer structure wherein a third layer containing a therapeutic agent, is positioned between a first layer containing a degradable polymer and a second layer containing the same or a different degradable polymer. The third layer may comprise a degradable polymer and a therapeutic agent, whereas the first and second layer may comprise only a degradable polymer. For example, the first and second layers may comprise a copolymer of about 10 weight % glycolide and about 90 weight % ε-caprolactone; while the third layer comprises a copolymer of about 10 weight % glycolide and about 90 weight % ε -caprolactone in combination with bupivacaine hydrochloride. In embodiments, the therapeutic agent to degradable polymer ratio is from about 5 to about 3.

The tri-layer structure, similar to a sandwich-type structure, may be combined with other films including other single, double, and/or other tri-layer structures.

FIG. 6B shows multilayer film 10B displaying tri-layer structure 12 including first layer 24, second layer 26, and third layer 28 with the therapeutic agent positioned between two polymer layers. First layer 24 may include at least one polymer and second layer 26 may include at least one therapeutic agent. Third layer 28 may include the same or different polymer as included in first layer 24. For example, in some embodiments, second layer 26 may include a therapeutic agent, such as bupivacaine, and the first and third layers may include the same polymer material, i.e., poly(glycolide-co-caprolactone). In another example, all three layers may include the same polymer material, i.e., poly(glycolide-co-caprolactone) and second layer 26 may also include a therapeutic agent, i.e., bupivacaine.

FIG. 6C illustrates two tri-layer structure 12A and 12B as shown in FIG. 6B stacked on top of each other to form multilayer film 10C. First tri-layer structure 12A includes first layer 24A, second layer 26A, and third layer 28A with the therapeutic agent positioned in second layer 26A between the two polymer layers, first and third layers 24A and 28A. Second tri-layer structure 12B includes fourth layer 24B, fifth layer 26B, and sixth layer 28B with the therapeutic agent positioned in fifth layer 26B between the two polymer layers, fourth and sixth layers 24A and 28A. Although shown as a sandwich-like structure in FIG. 6B, tri-layer structures 12A and 12B may include any conceivable combination of the polymer materials and therapeutic agents described herein.

When stacked, film 10 may include increased payload of the therapeutic agent without compromising mechanical properties. It is envisioned that more than two tri-layer structures 12 may be stacked on top of each other to form the films. In embodiments, 2 to about 25 of the tri-layer structure may be stacked on top of each other to form the film.

In embodiments, the multilayer film includes a tri-layer structure such as in FIG. 6B however the second, middle layer may not extend to the outer edge of the film. Rather, at least one of the first layer and third layers may be positioned along the outer edge and covering the second layer. By controlling the distance of the second layer (containing the therapeutic agent) to the outer edge the release of the therapeutic agent may be altered/controlled.

In certain embodiments, at least one of the first layer and third layers may cover the second layer along the entire length of the outer edge. In other embodiments, at least one of the first layer and third layers may cover the second layer along only intermittent portions of the length of the outer edge to create intermittent portions along the outer edge wherein the second layer is not covered (not shown).

Multilayer films of the present disclosure may be sprayed onto an inert substrate which may include a release liner substrate utilized for fabrication only. The inert substrate may be separated from the film prior to packaging or conversely, the substrate may be packaged with the film and removed prior to implantation. In other embodiments, at least a portion of a medical device, e.g., surgical mesh, may be positioned on an inert substrate, and the multilayer film may be sprayed directly onto the mesh, creating a multilayer composite film. In other embodiments, the inert substrate is part of the implantable therapeutic multilayer composite film.

The medical device may be positioned within any portion of the films described herein. For example, the films may be formed on an outer surface of the medical device. In some embodiments, the medical devices may be embedded in the films described herein.

In embodiments, the films of the present disclosure may be combined with a medical device using methods including but not limited to adhesives, glues, solvent welding, spot welding, solvent casting, melt pressing, heat staking, and the like. In other embodiments, the multilayer film may be formed directly on the medical device.

In embodiments, the configurations of the films of the present disclosure may include circular configurations, oval configurations, U-bend configurations, square configurations having a circular aperture, wave configurations, and irregular shape configurations. One of ordinary skill in the art will appreciate that the specific shape or configuration of film 10 can vary as desired and that the shapes and configurations described are illustrative of only a small number of possible shapes and configuration.

In some embodiments, the films include a single layer containing a degradable polymer and a therapeutic agent. Turning now to FIG. 5, film 10 contains at least one degradable polymer and at least one therapeutic agent in a single layer. Film 10 maintains flexibility to the extent it can be handled without tearing prior to implantation and can adjust to various amounts of force when implanted.

The films described herein may display a tensile strength sufficient to maintain a predetermined configuration. For example, in embodiments, the films may form a generally planar configuration displaying a mechanical strength sufficient to maintain the film in the generally planar configuration before, during or after implantation. Although the films do not require an additional substrate for support or to maintain the predetermined configuration, it is envisioned that in some embodiments the films described herein may also be combined with any implantable medical device as mentioned above. In embodiments, the films of the present disclosure may be utilized with meshes, buttresses, and tissue scaffold which may be at least partially in contact with or embedded within the film.

The film thickness may be controlled by factors such as the number of applications of the first and second solutions (described above), the length of time/rate spraying the first and second solutions, polymer solution composition, drug solution composition, flow rate, and use of additives such as viscosity modifiers. However, the thickness of the films described herein may on average measure between about 10 µm to about 3000 µm. In some embodiments, the thickness of the films may measure between about 20 µm to about 1000 µm. In still other embodiments, the thickness of the films may measure between about 25 µm to about 500 µm. Film thickness may influence parameters such as mechanical strength of the multilayer films.

Film thickness may also play a role in the drug release and diffusion. In embodiments, the thickness of each of the individual layers in the multilayer films may control the release of the therapeutic agent from the film. For example, it has been shown that as film thickness increases, the rate of release for the therapeutic agent decreases or slows down over time. Conversely, decreasing the thickness of only the polymer layer may increase the rate at which the therapeutic agent may be released.

Tissue reactive chemistries may also be added to the multilayered films of the present disclosure. Suitable tissue reactive chemistries include, for example, reactive silicones, isocyanates, N-hydroxy succinimides ("NHS"), cyanoacrylates, aldehydes (e.g., formaldehydes, glutaraldehydes, glyceraldehydes, and dialdehydes), and genipin. As used herein, succinimides also include sulfosuccinimides, succinimide esters and sulfosuccinimide esters, including N-hydroxysuccinimide ("NHS"), N-hydroxysulfosuccinimide ("SNHS"), N-hydroxyethoxylated succinimide ("ENHS"), N-hydroxysuccinimide acrylate, succinimidyl glutarate, n-hydroxysuccinimide hydroxybutyrate, combinations thereof, and the like.

The compositions and methods of forming a film provided in the present disclosure may produce films for treating post-operative pain. Post-operative pain may be commonly associated with procedures such as hernia repair, e.g., inguinal and ventral hernias, a hysterectomy, a thoracotomy, coronary artery bypass, a hemorrhoidectomy, adhesiolysis, breast reconstruction, spine surgery, cosmetic surgery, dental surgery, tissue biopsies and joint repair/replacement. Suitable therapeutic agents which may be used in treating post-operative pain include, but are not limited to bupivacaine hydrochloride, lidocaine hydrochloride, mepivacaine hydrochloride, capsaicin and combinations thereof.

The compositions and methods of forming a film provided in the present disclosure may produce films for treating cancers not limited to those such as breast cancer, pancreatic cancer, liver cancer, lung cancer, esophageal cancer, gastric cancer, colon cancer, stomach cancer, and brain cancer. Suitable therapeutic agents which may be used in treating cancer include, but are not limited to 5-fluorouracil, methotrexate, cisplatin, daunorubucub, mitoxantrone, and carboplatin.

The following Examples are being submitted to illustrate embodiments of the present disclosure. These Examples are intended to be illustrative only and are not intended to limit the scope of the present disclosure. Also, parts and percentages are by weight unless otherwise indicated.

### EXAMPLES

### COMPARATIVE EXAMPLE 1

A first polymer solution and a second therapeutic solution were prepared. The first polymer solution (about 3% w/v) was prepared including, a glycolide-ε-caprolactone copolymer, and dichloromethane. A second therapeutic solution of bupivacaine HCl (about 5% w/v) and polymer solution (about 3% w/v) was prepared in a 1:1 mixture of dichloromethane and ethanol. A multi-laminar film was prepared by first spraying the first polymer solution onto a removable silicone backing. The solution was sprayed using about 48 kHz ultrasonic nozzle at a flow rate of about 2 mL/min, a power of about 2.25 W, and a nozzle speed of about 100 mm/s. The coating machine was programmed to traverse back and forth across the silicone substrate with an offset of about 10 mm after each movement. The spraying application was repeated about 14 times. Next, the second therapeutic solution was sprayed using the same conditions as the polymer solution. The spraying application was repeated about 250 times. Finally, the polymer solution was again applied about 14 times to the top of the structure. After drying, the silicone backing was removed. An SEM of the film produced magnified 150x is illustrated in FIG. 1. As shown, the resulting multi-laminar film was not a smooth film but rather an uneven, coral like structure with poor handling characteristics.

### EXAMPLE 1

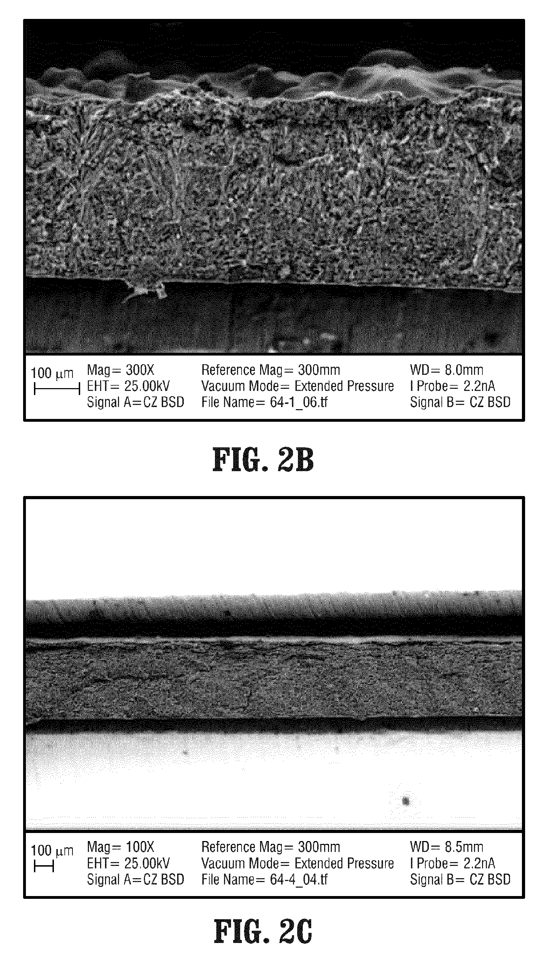

A first polymer solution (about 3% w/v) including a glycolide-ε-caprolactone copolymer, and dichloromethane was prepared. A second therapeutic solution of bupivacaine HCl (about 5% w/v) and polymer solution (about 3% w/v) was prepared in about 1:1 mixture of dichloromethane and methanol respectively. A multi-laminar film was prepared by first spraying the polymer solution onto a removable silicone backing. The solution was sprayed using about 48 kHz ultrasonic nozzle at a flow rate of about 2 mL/min, a power of about 2.25 W, and a nozzle speed of about 100 mm/s. The coating machine was programmed to traverse back and forth across the silicone substrate with an offset of about 10 mm after each movement. The spraying application was repeated about 14 times. Next, the therapeutic solution was sprayed using the same conditions as the polymer solution. The spraying application was repeated about 250 times to create a film with a payload of about 20 mg/cm2 bupivacaine HCl. Finally, the polymer solution was again applied about 14 times to the top of the film. After drying, the silicone backing was removed. A linear release profile was observed and total release occurred between about 48 and about 72 hours. An SEM of the film magnified 100x is illustrated in FIG. 2A. An SEM of the film produced magnified 300x is illustrated in FIG. 2B. As shown, the resulting multi-laminar film was smoother with good handling characteristics and preferred release kinetics.

### EXAMPLE 2

A first polymer solution (about 3% w/v) including a glycolide-ε-caprolactone copolymer, and dichloromethane was prepared. A second therapeutic solution of bupivacaine HCl (about 5% w/v) and polymer solution (about 3% w/v) was prepared in about a 4:1 mixture of dichloromethane and methanol respectively. A multi-laminar film was prepared by first spraying the polymer solution onto a removable silicone backing. The solution was sprayed using about 48 kHz ultrasonic nozzle at a flow rate of about 2 mL/min, a power of about 2.25 W, and a nozzle speed of about 100 mm/s. The coating machine was programmed to traverse back and forth across the silicone substrate with an offset of about 10 mm after each movement. The spraying application was repeated about 14 times. Next, the therapeutic solution was sprayed using the same conditions as the polymer solution. The spraying application was repeated about 250 times to create a film with a payload of about 20 mg/cm2 bupivacaine HCl. Finally, the polymer solution was again applied about 14 times to the top of the film. After drying, the silicone backing was removed. A linear release profile was observed and total release occurred between about 48 and about 72 hours. An SEM of the film magnified 100x is illustrated in FIG. 2C. An SEM of the film produced magnified 600x is illustrated in FIG. 2D. As shown, the resulting multi-laminar film was smoother with desired handling characteristics and preferred release kinetics.

In Fig. 2E, the surface smoothness was measured by extending an imaginary line at the highest peak of the outer surface and generally parallel following the contour of the outer surface of the film and measuring the area (Rₐ) between the extended parallel line and the outer surface of the film. As shown, at 600X magnification, the Rₐ for samples fabricated using 1:4 ratio of methanol to dichloromethane was 2087 µm².

### EXAMPLE 3

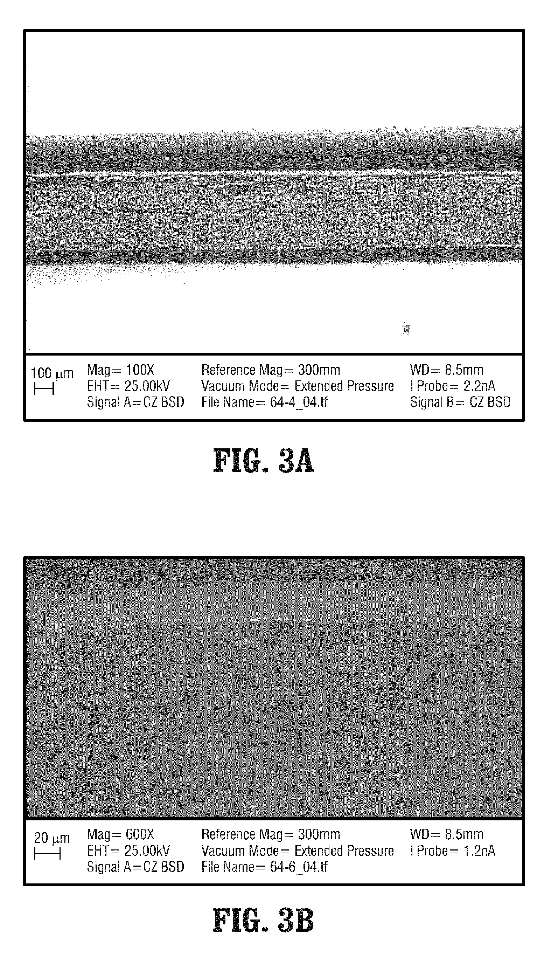

A first polymer solution (about 3% w/v) including a glycolide-ε-caprolactone copolymer, and dichloromethane was prepared. A second therapeutic solution of bupivacaine HCl (about 5% w/v) and polymer solution (about 3% w/v) was prepared in about a 9:1 mixture of dichloromethane and methanol respectively. A multi-laminar film was prepared by first spraying the polymer solution onto a removable silicone backing. The solution was sprayed using about 48 kHz ultrasonic nozzle at a flow rate of about 2 mL/min, a power of about 2.25 W, and a nozzle speed of about 100 mm/s. The coating machine was programmed to traverse back and forth across the silicone substrate with an offset of about 10 mm after each movement. The spraying application was repeated about 14 times. Next, the therapeutic solution was sprayed using the same conditions as the polymer solution. The spraying application was repeated about 250 times to create a film with a payload of about 20 mg/cm2 bupivacaine HCl. Finally, the polymer solution was again applied about 14 times to the top of the film. After drying, the silicone backing was removed. A linear release profile was observed and total release occurred between about 48 and about 72 hours. An SEM of the film magnified 100x is illustrated in FIG. 3A. An SEM of the film produced magnified 600x is illustrated in FIG. 3B. As shown, the resulting multi-laminar film was smoother with desired handling characteristics and preferred release kinetics.

Although not depicted, at 600X magnification, the Rₐ for samples fabricated using a 1:9 ratio of methanol to dichloromethane was 2136 µm². Samples fabricated using a 1:1 ratio of methanol to dichloromethane, at 600X magnification displayed a Rₐ of about 3957 µm².

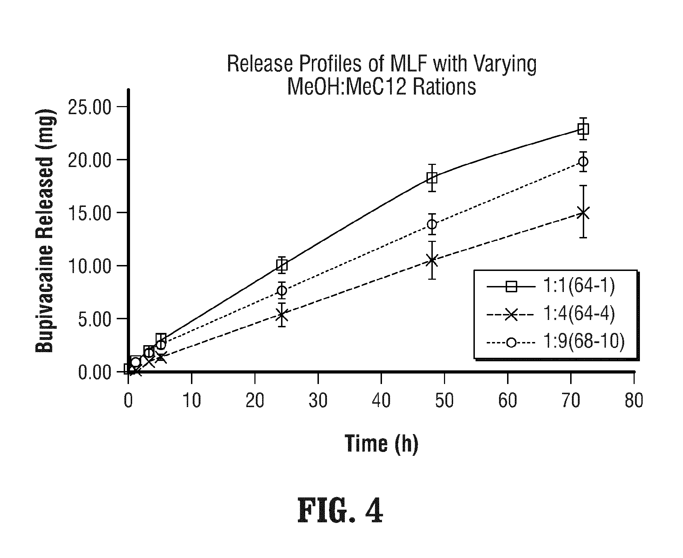

Release profiles of the multi-laminar films with varying methanol to dichloromethane ratios of Examples 1-3 are illustrated in FIG. 4. The multi-laminar films including about 1:4 to about 1:9 ratios of methanol to dichloromethane produces smoother films with linear release profiles that were easier to reproduce. The smoother films were more stable, especially at temperatures during ethylene oxide sterilization.

It will be understood that various modifications may be made to the embodiments disclosed herein. Therefore, the above description should not be construed as limiting, but merely as exemplifications within the scope and spirit of the claims appended hereto.

The invention may be described by reference to the following numbered paragraphs:-
1. A method of forming a film comprising:
   providing a first solution containing methanol, a chlorinated hydrocarbon and at least one therapeutic agent, wherein the ratio of methanol to the chlorinated hydrocarbon is from about 1:1 to about 1:20;
   providing a second solution containing at least one polymer;
   combining the first and second solution to form a film-forming composition; and
   forming at least one layer of the film with the film-forming composition.
2. The method of paragraph 1, wherein the ratio of the methanol to the chlorinated hydrocarbon is from about 1:3 to about 1:10.
3. The method of paragraph 1, wherein the ratio of the methanol to the chlorinated hydrocarbon is from about 1:4 to about 1:9.
4. The method of paragraph 1, wherein the ratio of the methanol to the chlorinated hydrocarbon is from about 1:9.
5. The method of paragraph 1, wherein the chlorinated hydrocarbon comprises a lower boiling point than methanol.
6. The method of paragraph 1, wherein the at least one chlorinated hydrocarbon is selected from the group consisting of methyl chloride, dichloromethane, chloroform, carbon tetrachloride, and combinations thereof.
7. The method of paragraph 1, wherein the at least one chlorinated hydrocarbon is dichloromethane.
8. The method of paragraph 1, wherein the at least one therapeutic agent is selected from the group consisting of salt forms of bupivacaine, fluorouracil, cisplatin, methotrexate, capsaicin, and combinations thereof.
9. The method of paragraph 1, wherein the at least one therapeutic agent is bupivacaine hydrochloride.
10. The method of paragraph 1, wherein the at least one polymer is derived from a monomer selected from the group consisting of lactide; glycolide; trimethylene carbonate; caprolactone; Δ-valerolactone; β-butyrolactone; γ-butyrolactone; ε-decalactone; hydroxybutyrate; hydroxyvalerate; 1,4-dioxepan-2-one; 1,5-dioxepan-2-one; 6,6-dimethyl- 1,4-dioxan-2-one; 2,5-diketomorpholine; pivalolactone; α, α diethylpropiolactone; ethylene carbonate; ethylene oxalate; 3-methyl-1,4-dioxane-2,5-dione; 3,3-diethyl-1,4-dioxan-2,5-dione; 6,8-dioxabicycloctane-7-one; and homopolymers and copolymers and combinations thereof.
11. The method of paragraph 1, wherein the at least one polymer comprises a copolymer of about 10 weight % glycolide and about 90 weight % ε-caprolactone.
12. The method of paragraph 1, further comprising depositing a barrier layer on the at least one layer of the film.
13. The method of paragraph 1, further comprising sterilizing the film with ethylene oxide.
14. The method of paragraph 1, wherein the film is formed by spray coating.
15. The method of paragraph 14, wherein the spray coating comprises ultrasonic spray coating.
16. The method of paragraph 1, wherein the film displays a linear release profile of the therapeutic agent.
17. A method of forming a film comprising:
   providing a composition containing methanol, at least one chlorinated solvent, at least one therapeutic agent, and at least one polymer, wherein the ratio of the methanol to the at least one chlorinated solvent is from about 1:1 to about 1:20; and
   forming at least one layer of the film with the composition.
18. A drug-eluting film formed by the method of paragraph 17.
19. A composition for forming a film comprising:
   a therapeutic agent;
   a polymer;
   an alcohol; and
   a chlorinated hydrocarbon; wherein the ratio of the alcohol to the at least one chlorinated hydrocarbon is from about 1:4 to about 1:9.

## Claims

1. A method of forming a film comprising:
providing a composition containing methanol, at least one chlorinated solvent, at least one therapeutic agent, and at least one polymer, wherein the ratio of the methanol to the at least one chlorinated solvent is from about 1:1 to about 1:20; and
forming at least one layer of the film with the composition.

2. A method according to claim 1, comprising:
providing a first solution containing methanol, a chlorinated hydrocarbon and at the least one therapeutic agent, wherein the ratio of methanol to the chlorinated hydrocarbon is from about 1:1 to about 1:20;
providing a second solution containing the at least one polymer;
combining the first and second solution to form a film-forming composition; and
forming at least one layer of the film with the film-forming composition.

3. The method of claim 1 or claim 2, wherein the ratio of the methanol to the chlorinated hydrocarbon is from about 1:3 to about 1:10.

4. The method of claim 3, wherein the ratio of the methanol to the chlorinated hydrocarbon is from about 1:4 to about 1:9.

5. The method of claim 4, wherein the ratio of the methanol to the chlorinated hydrocarbon is from about 1:9.

6. The method of any preceding claim, wherein the chlorinated hydrocarbon comprises a lower boiling point than methanol.

7. The method of any preceding claim, wherein the at least one chlorinated hydrocarbon is selected from the group consisting of methyl chloride, dichloromethane, chloroform, carbon tetrachloride, and combinations thereof; preferably, wherein the at least one chlorinated hydrocarbon is dichloromethane.

8. The method of any preceding claim, wherein the at least one therapeutic agent is selected from the group consisting of salt forms of bupivacaine, fluorouracil, cisplatin, methotrexate, capsaicin, and combinations thereof; preferably, wherein the at least one therapeutic agent is bupivacaine hydrochloride.

9. The method of any preceding claim, wherein the at least one polymer is derived from a monomer selected from the group consisting of lactide; glycolide; trimethylene carbonate; caprolactone; Δ-valerolactone; β-butyrolactone; γ-butyrolactone; ε-decalactone; hydroxybutyrate; hydroxyvalerate; 1,4-dioxepan-2-one; 1,5-dioxepan-2-one; 6,6-dimethyl- 1,4-dioxan-2-one; 2,5-diketomorpholine; pivalolactone; α, α diethylpropiolactone; ethylene carbonate; ethylene oxalate; 3-methyl-1,4-dioxane-2,5-dione; 3,3-diethyl-1,4-dioxan-2,5-dione; 6,8-dioxabicycloctane-7-one; and homopolymers and copolymers and combinations thereof.

10. The method of claim 9, wherein the at least one polymer comprises a copolymer of about 10 weight % glycolide and about 90 weight % ε-caprolactone.

11. The method of any preceding claim, further comprising depositing a barrier layer on the at least one layer of the film.

12. The method of any preceding claim, further comprising sterilizing the film with ethylene oxide.

13. The method of any preceding claim, wherein the film is formed by spray coating, preferably wherein the spray coating comprises ultrasonic spray coating.

14. The method of any preceding claim, wherein the film displays a linear release profile of the therapeutic agent.

15. A drug-eluting film formed by the method of any preceding claim.

16. A composition for forming a film comprising:
a therapeutic agent;
a polymer;
an alcohol; and
a chlorinated hydrocarbon; wherein the ratio of the alcohol to the at least one chlorinated hydrocarbon is from about 1:4 to about 1:9.
